# EUROPEAN PATENT APPLICATION

(11) **EP 4 841 642 A1**
(43) Date of publication of application: **18.11.2026**
(21) Application number: 25215198.0
(22) Date of filing: 12.11.2025
(51) Int. Cl.: G16H 20/30, G06F 3/01, G16H 20/70, G16H 50/30

(54) **VIRTUAL REALITY-BASED VESTIBULAR REHABILITATION TRAINING SYSTEM AND METHOD**

(30) Priority: 14.05.2025 KR 20250062801
(71) Applicant: NeuroEars Co., Ltd., Chuncheon-si, Gangwon-do 24232 (KR); Industry Academic Cooperation Foundation, Hallym University, Chuncheon-si, Gangwon-do 24252 (KR)
(72) Inventor: HONG, Sung Kwang, 13600 Seongnam-si, (KR); WANG, Zong Sheng, 24362 Chuncheon-si (KR); YOON, Dae Hee, 15840 Gunpo-si (KR)
(74) Representative: Reich, Jochen

(57) **Abstract**

The present disclosure discloses a virtual reality-based vestibular rehabilitation training system and method. According to an embodiment of the present disclosure, the vestibular rehabilitation training system may include a vestibular rehabilitation training control device and a vestibular rehabilitation training performing device. The vestibular rehabilitation training control device may input questionnaire information of a patient into a large language model to generate training setting information, and transmit the training setting information to the vestibular rehabilitation training performing device. The vestibular rehabilitation training performing device may output vestibular rehabilitation training content based on the training setting information, track the patient's gaze and head movements to calculate a training evaluation score, and transmit the training evaluation score to the vestibular rehabilitation training control device.

## Description

### BACKGROUND

### Field

The present disclosure relates to a vestibular rehabilitation training system and method, and more particularly, to a virtual reality-based vestibular rehabilitation training system and method.

### Description of the Related Art

The content described in this section merely provides background information on the embodiments described in the present disclosure and does not necessarily constitute prior art.

Vestibular rehabilitation therapy is a treatment method that improves the symptoms of dizziness patients by using head and eye movements. Vestibular rehabilitation therapy is widely used in clinical practice because the effectiveness has been proven in almost all types of dizziness patients from an evidence-based medicine perspective. However, despite the excellent effects of vestibular rehabilitation therapy, there is a problem that actual patients may not faithfully participate in the treatment due to tedious problems in the method.

### SUMMARY

An object of the present disclosure is to provide a virtual reality-based vestibular rehabilitation training system and method.

The present disclosure is not limited to the objects mentioned above, and other objects not mentioned may be clearly understood by those skilled in the art from the description below.

According to the present disclosure for achieving the above-described objects, there is provided a vestibular rehabilitation training control device, including: a hardware processor; and a storage unit connected to the processor and storing a database storing questionnaire information, clinical information, and rehabilitation training information of a plurality of patients as a data set, and storing at least one computer program configured to perform a vestibular rehabilitation training control method, in which the vestibular rehabilitation training control method includes (a) training a large language model to generate training setting information by receiving the questionnaire information using the questionnaire information, clinical information, and rehabilitation training information of the plurality of patients as learning data, (b) comparing the questionnaire information input from the patient with a plurality of data sets stored in the database to generate the training setting information or inputting the questionnaire information to the large language model to obtain the training setting information, and (c) transmitting the training setting information to a vestibular rehabilitation training performing device.

According to one embodiment of the present disclosure, the questionnaire information may include Dizziness Handicap Inventory (DHI) and Vestibular Disorders Activities of Daily Living Scale (VADL) questionnaire information.

According to one embodiment of the present disclosure, the (b) may include generating, when a data set having the same questionnaire information as the questionnaire information input from the patient exists in the database, the training setting information based on rehabilitation training information of the data set, and inputting, when the data set having the same questionnaire information as the questionnaire information input from the patient does not exist in the database, the questionnaire information into the large language model to obtain the training setting information.

According to one embodiment of the present disclosure, the training setting information may include at least one of a vestibular rehabilitation training content type and a training difficulty level.

According to one embodiment of the present disclosure, the vestibular rehabilitation training control method may further include (d) modifying the training setting information based on a training evaluation score received from the vestibular rehabilitation training performing device.

According to one embodiment of the present disclosure, the (d) may include setting the training difficulty level included in the training setting information to be relatively low when the training evaluation score is equal to or less than a preset reference value.

According to the present disclosure for achieving the above-described object, there is provided a vestibular rehabilitation training performing device including: a housing having a preset shape, and including a display unit for displaying vestibular rehabilitation training content, an eye tracker for tracking the gaze of a patient, and an Inertial Measurement Unit (IMU) sensor for sensing a head movement of the patient; a storage unit for storing at least one vestibular rehabilitation training content and at least one computer program configured to perform a vestibular rehabilitation training performing method; a communication unit for receiving training setting information from a vestibular rehabilitation training control device and transmitting a training evaluation score to the vestibular rehabilitation training control device; a training progress unit for outputting the vestibular rehabilitation training content based on the training setting information received from the vestibular rehabilitation training control device, generating position information of a gaze point according to the gaze of the patient using data tracked by the eye tracker, and generating head movement information of the patient using data sensed by the IMU sensor to perform the vestibular rehabilitation training content; and a score calculation unit for calculating the training evaluation score for the vestibular rehabilitation training content using the position information of the gaze point and the head movement information.

According to one embodiment of the present disclosure, the at least one vestibular rehabilitation training content may include at least one of vestibular ocular reflex exercise training content, active eye-head exercise training content, and target imagination exercise training content.

According to one embodiment of the present disclosure, when the vestibular rehabilitation training content is vestibular ocular reflex exercise training content, the training progress unit may output a virtual target having a preset size and fixed or moving at an arbitrary location, and determine whether the gaze point is located in an area occupied by the virtual target when the patient fixes or moves the head.

In this case, the score calculation unit may calculate the training evaluation score in proportion to the number of times that the gaze point is located in the area occupied by the virtual target.

According to another embodiment of the present disclosure, when the vestibular rehabilitation training content is the active eye-head movement training content, the training progress unit may output two virtual targets having preset sizes at different locations, and when the patient moves the patient's gaze toward a first virtual target and then turns the patient's head toward the first virtual target, the training progress unit may determine whether the gaze point is located in an area occupied by the first virtual target, and when the patient moves the patient's gaze toward a second virtual target and then turns the patient's head toward the second virtual target, the training progress unit may determine whether the gaze point is located in an area occupied by the second virtual target.

In this case, when the patient turns the patient's head toward the first virtual target or the second virtual target, the score calculation unit may calculate the training evaluation score in inverse proportion to a time taken for the gaze point to move back to the area occupied by the virtual target after deviating the area occupied by the virtual target.

According to another embodiment of the present disclosure, when the vestibular rehabilitation training content is the target imagination exercise training content, the training progress unit may output a virtual target having a preset size and fixed at an arbitrary location, and when the patient closes the patient's eyes in a state where the patient's gaze is directed toward the virtual target and the patient opens the patient's eyes after turning the patient's head, the training progress unit may determine whether the gaze point is located in the area occupied by the virtual target.

In this case, the score calculation unit may calculate the training evaluation score in inverse proportion to an extent to which the gaze point deviates from the area occupied by the virtual target.

According to one embodiment of the present disclosure, the training progress unit may provide visual feedback when the gaze point is located in an area where at least one virtual target output from the vestibular rehabilitation training content is located.

The vestibular rehabilitation training control device and the vestibular rehabilitation training performing device according to an embodiment of the present disclosure may be a component of a vestibular rehabilitation training system.

The vestibular rehabilitation training control method according to the embodiment of the present disclosure may be implemented in the form of a computer program prepared to perform each step on a computer and recorded on a computer-readable recording medium.

Other specific details of the present disclosure are included in the detailed description and drawings.

According to one aspect of the present disclosure, the vestibular rehabilitation training system can provide customized rehabilitation training content to the patient based on the results of the questionnaire information input by the patient, and can quantitatively calculate training performance to adjust difficulty of the rehabilitation training. Through this, the patient can receive more precise and effective rehabilitation training than before through the vestibular rehabilitation training system.

According to another aspect of the present disclosure, the vestibular rehabilitation training system can provide real-time feedback to a patient to encourage the patient to actively perform vestibular rehabilitation training.

Effects of the present disclosure are not limited to the effects mentioned above, and other effects not mentioned will be clearly understood by those skilled in the art from the description below.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

### BRIEF DESCRIPTION OF DRAWINGS

The above and other aspects, features and other advantages of the present disclosure will be more clearly understood from the following detailed description taken in conjunction with the accompanying drawings, in which:
FIG. 1 is a block diagram of a vestibular rehabilitation training system according to one embodiment of the present disclosure;
FIG. 2 is a flow diagram of a vestibular rehabilitation training control method and a vestibular rehabilitation training performing method according to one embodiment of the present disclosure;
FIG. 3 is a diagram illustrating an example of training setting information generated based on questionnaire information, (a) of FIG. 3 is an example of questionnaire items provided to a patient, and (b) of FIG. 3 is an example of information generated by a large language model;
FIG. 4 is a block diagram of a vestibular rehabilitation training performing device according to one embodiment of the present disclosure.
FIGS. 5A to 5C is diagrams illustrating examples of vestibular ocular reflex exercise training content according to one embodiment of the present disclosure. FIG. 5A is an example of first vestibular ocular reflex exercise training content, and FIG. 5B is an example of second vestibular ocular reflex exercise training content, and FIG. 5C is an example of third vestibular ocular reflex exercise training content;
FIG. 6 is a diagram illustrating an example of an error tolerance range according to a training difficulty level;
FIG. 7 is a diagram illustrating an example of active eye-head exercise training content;
FIG. 8 is a diagram illustrating an example of target imagination exercise training content;
FIG. 9 is a diagram illustrating an example of focusing exercise training content; and
FIG. 10 is a flowchart of a vestibular rehabilitation training control method and a vestibular rehabilitation training performing method according to another embodiment of the present disclosure.

### DETAILED DESCRIPTION OF THE EMBODIMENT

Hereinafter, the exemplary embodiment of the present disclosure will be described with reference to the accompanying drawings and exemplary embodiments as follows. Scales of components illustrated in the accompanying drawings are different from the real scales for the purpose of description, so that the scales are not limited to those illustrated in the drawings.

The advantages and features of the disclosure disclosed in the present disclosure, and the methods for achieving them, will become apparent by referring to the embodiments described in detail below together with the accompanying drawings. However, the present disclosure is not limited to the embodiments disclosed below and may be implemented in various different forms, and the present embodiments are provided only to make the disclosure of the present disclosure complete and to fully inform those skilled in the art (hereinafter referred to as "ordinary skilled in the art") of the scope of the present disclosure, and the scope of rights of the present disclosure is defined only by the scope of the claims.

The terminology used in the present disclosure is for the purpose of describing embodiments only and is not intended to limit the scope of the present disclosure. In the present disclosure, the singular also includes the plural unless specifically stated otherwise. The terms "comprise" and/or "comprising" as used herein do not exclude the presence or addition of one or more other components in addition to the components mentioned.

Throughout the disclosure, the same reference numerals refer to the same elements, and "and/or" includes each and every combination of one or more of the elements mentioned. Although "first", "second", or the like are used to describe various elements, it is to be understood that these elements are not limited by these terms. These terms are merely used to distinguish one element from another. Accordingly, it should be understood that a first element mentioned below may also be a second element within the technical scope of the present disclosure.

Unless otherwise defined, all terms (including technical and scientific terms) used in the present disclosure may be used in the meaning commonly understood by those skilled in the art to which the present disclosure belongs. In addition, terms defined in commonly used dictionaries shall not be ideally or excessively interpreted unless explicitly specifically defined.

An artificial neural network (ANN) is an artificial intelligence system that implements artificial intelligence by connecting artificial neurons obtained by mathematically modelling the neurons that constitute the human brain.

In the present disclosure, an "artificial neural network model" may be composed of a set of interconnected computational units, which may generally be referred to as nodes. These nodes may also be referred to as neurons.

In a neural network, one or more nodes connected through a link may form a relationship of input node and output node relatively. The concept of input node and output node is relative, and any node in an output node relationship to one node may be in an input node relationship in a relationship to another node, and vice versa. As described above, the input node-to-output node relationship may be created based on a link. One or more output nodes may be connected to one input node through a link, and vice versa.

In the present disclosure, "inputting" data into the artificial neural network model means that a value is input to the first input node. In the present disclosure, "obtaining a value," "outputting data," "obtaining information" from an artificial neural network means that some data is output from the last output node.

In the present disclosure, "learning" of an artificial neural network model means updating the connection weights of each node so that the error in the output is minimized, and "learning" according to the present disclosure is not limited by a specific learning method.

In the present disclosure, a "processor" may be composed of one or more cores, and may include a processor for data analysis and deep learning, such as a central processing unit (CPU), a general-purpose graphics processing unit (GPGPU), and a tensor processing unit (TPU) of a computing device. The processor may read a computer program stored in a memory and perform data processing for machine learning according to one embodiment of the present disclosure.

Hereinafter, embodiments of the present disclosure will be described in detail with reference to the attached drawings.

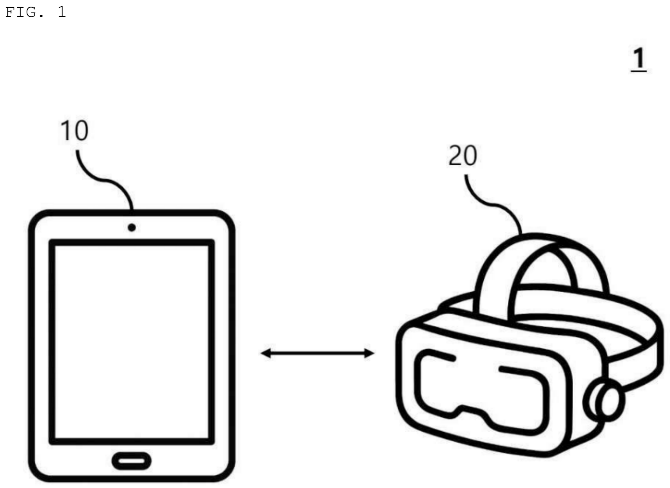

FIG. 1 is a block diagram of a vestibular rehabilitation training system according to one embodiment of the present disclosure.

Referring to FIG. 1, the vestibular rehabilitation training system according to one embodiment of the present disclosure may include a vestibular rehabilitation training control device 10 and a vestibular rehabilitation training performing device 20.

The vestibular rehabilitation training control device 10 according to one embodiment of the present disclosure may include a hardware processor and a storage unit connected to the processor.

The hardware processor may include a general-purpose processor, an application-specific integrated circuit (ASIC), another chipset, a logic circuit, a register, a communication modem, a data processing device, or the like known in the art to which the present disclosure pertains to execute calculations and various control logics. In addition, when the logic to be described below is implemented as software, the software may be stored in a memory device and executed by the processor.

The storage unit may store a database storing questionnaire information, clinical information and rehabilitation training information of a plurality of patients as a data set, and at least one computer program configured to perform a vestibular rehabilitation training control method.

The database may store each patient's questionnaire information, clinical information, and rehabilitation training information as separate data sets.

The vestibular rehabilitation training control device 10 may be implemented using various computing devices such as tablet computers, smartphones, computers, and laptops.

The above questionnaire information may include the Dizziness Handicap Inventory (DHI) and the Vestibular Disorders Activities of Daily Living Scale (VADL) questionnaire information. The questionnaire information may include the total score, physical score, emotional score, and functional score of the questionnaire calculated based on the DHI and VADL. In addition, the questionnaire information may include the score for each item of the DHI and VADL. Since calculating the above-described scores from the DHI and VADL to determine vestibular function abnormality is a widely known technique among those skilled in the art, a detailed description thereof will be omitted.

The clinical information may include the contents regarding the patient's gender, age, and medical records according to vestibular dysfunction. For example, the medical records may include symptom information according to the vestibular dysfunction, such as situations in which the patient feels dizzy (when moving the head, when walking on the sidewalk, or the like) and emotional state due to dizziness.

The rehabilitation training information may include the contents about the type of vestibular rehabilitation training performed by the patient, the difficulty of the training, the number of rehabilitation training sessions, the evaluation score, or the like.

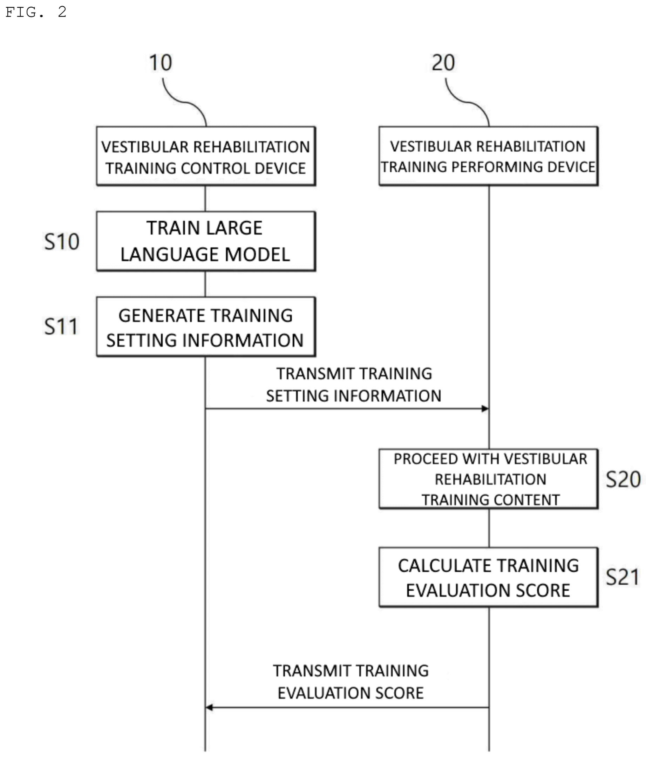

FIG. 2 is a flowchart of the vestibular rehabilitation training control method and the vestibular rehabilitation training performing method according to one embodiment of the present disclosure.

Referring to FIG. 2, in Step S10, the processor may train a large language model using the questionnaire information, clinical information, and rehabilitation training information of the plurality of patients as the learning data.

In the present disclosure, the "large language model (LLM)" refers to a very large deep learning model that has been pre-trained with a large amount of data. The basic transformer of LLM is a set of neural networks consisting of an encoder and a decoder with self-attention function. The encoder and decoder may extract meaning from a series of texts and understand the relationship between words and phrases in the text. The LLM includes OpenAI's ChatGPT, Google's Gemini (BARD), and Meta's LLaMA. In the present disclosure, the LLM is not limited to a specific model of a specific company, and any model that may perform the role of LLM described in the present disclosure is sufficient.

The processor may interact with the large language model through an Application Programming Interface (API) provided by the manufacturer of the large language model. The processor may input the above-described learning data in the form of a context through the API of the large language model. In this case, the processor may train the large language model to generate symptom information and training setting information according to an abnormality in the vestibular function based on the questionnaire information. Through this, the large language model may generate the symptom information and/or the training setting information using the patient's questionnaire information as an input value.

In Step S11, the processor may compare the questionnaire information input from the patient with a plurality of data sets stored in the database to generate the training setting information, or input the questionnaire information into the large language model to obtain training setting information.

More specifically, the processor may compare the questionnaire information input from the patient with the plurality of data sets stored in the database.

The processor may check whether there is a data set among the plurality of data sets having the same questionnaire information as the patient's questionnaire information. When there is the data set having the same questionnaire information as the patient's questionnaire information, the processor may generate training setting information based on the rehabilitation training information of the data set. In addition, the processor may generate the symptom information according to the vestibular dysfunction based on the clinical information of the data set.

When the data set having the same questionnaire information as the patient's questionnaire information does not exist in the database, the processor may input the patient's questionnaire information into the large language model to obtain the training setting information. In addition, the processor may obtain the symptom information according to the vestibular dysfunction from the large language model.

The same questionnaire information as the patient's questionnaire information may mean questionnaire information with the same score for each questionnaire item.

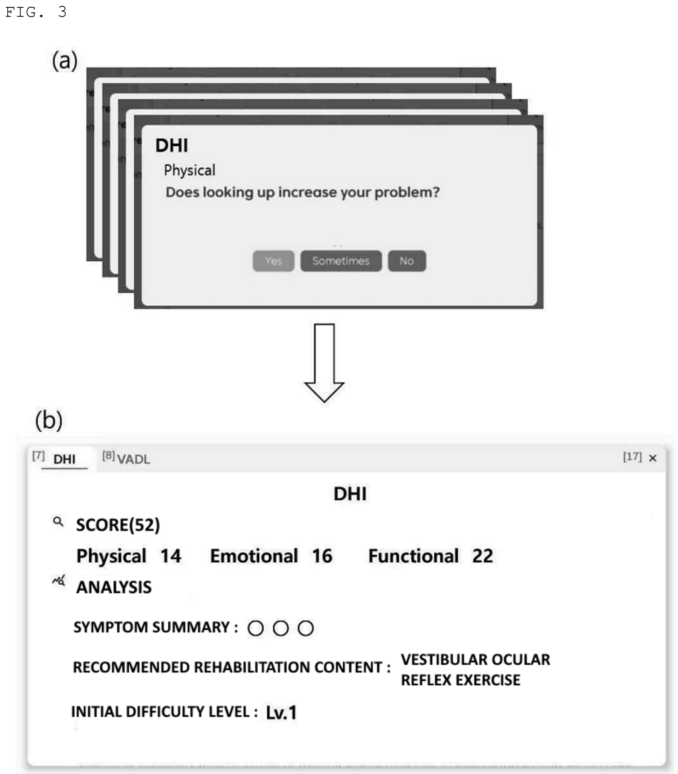

FIG. 3 is a diagram illustrating an example of the training setting information generated based on the questionnaire information. (a) of FIG. 3 is an example of the questionnaire items provided to the patient, and (b) of FIG. 3 is an example of information generated by the large language model.

Referring to FIG. 3, the patient may fill out a DHI and VADL questionnaire using the vestibular rehabilitation training control device 10 ((a) of FIG. 3). The processor may input the completed DHI and VADL questionnaire information into the large language model through the API of the large language model. Preferably, the processor may input a score list for each item of the DHI and VADL questionnaire into the large language model.

The large language model may receive DHI and VADL questionnaire information and generate the symptom information and training setting information according to the patient's vestibular dysfunction ((b) of FIG. 3).

The symptom information of the patient's vestibular dysfunction may include the intensity of the patient's dizziness, the occurrence of dizziness symptoms (when moving the head, when walking on the sidewalk, or the like), and physical, mental, and functional problems related to the dizziness.

The training setting information may include at least one of a vestibular rehabilitation training content type and a training difficulty level.

The vestibular rehabilitation training content may include at least one of vestibular ocular reflex exercise training content, active eye-head exercise training content, target imagination exercise training content, and focusing exercise training content. The large language model may select at least one of the contents based on DHI and VADL questionnaire information to generate the training setting information. The contents of each vestibular rehabilitation training content will be described in detail below.

Since the DHI and VADL questionnaire scores are proportional to the degree of vestibular dysfunction in the patient, the difficulty level of rehabilitation training may be set to be inversely proportional to the questionnaire scores. That is, the higher the DHI and VADL questionnaire scores, the lower the difficulty level of rehabilitation training may be set relatively. The contents related to the difficulty level of rehabilitation training will be explained in detail below.

The processor may store the patient's questionnaire information, the symptom information according to the vestibular dysfunction generated from the large language model, and the training setting information as a data set in the database.

The processor may transmit the training setting information to the vestibular rehabilitation training performing device 20. The vestibular rehabilitation training control device 10 and the vestibular rehabilitation training performing device 20 may transmit and receive data using wired/wireless communication. The patient may perform vestibular rehabilitation training according to the training setting information using the vestibular rehabilitation training performing device 20.

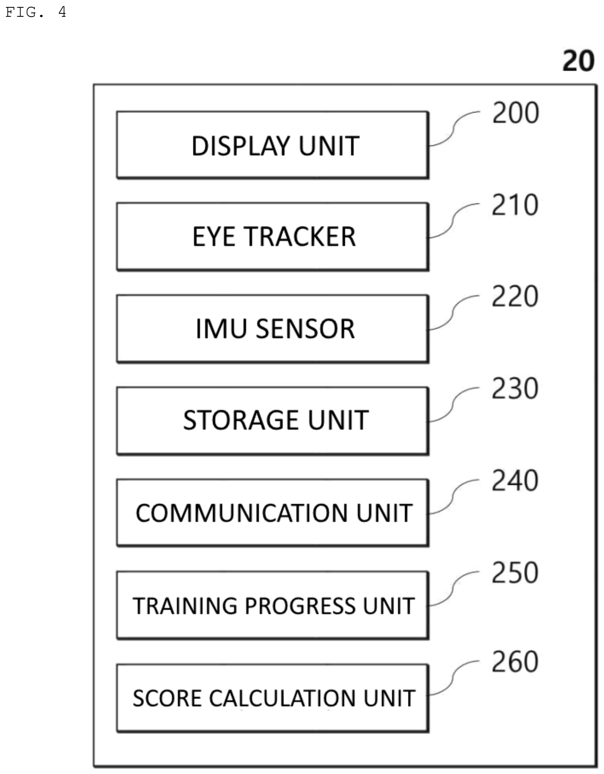

FIG. 4 is a block diagram of a vestibular rehabilitation training performing device according to one embodiment of the present disclosure.

Referring to FIGS. 1 and 4, the vestibular rehabilitation training performing device 20 may be implemented as a head mounted display (HMD) including a housing that may be worn on the patient's head. The patient may perform the vestibular rehabilitation training based on virtual reality, augmented reality, or mixed reality by wearing the vestibular rehabilitation training performing device 20.

The housing may include a display unit 200 that displays vestibular rehabilitation training content, an eye tracker 210 that tracks the patient's gaze, and an Inertial Measurement Unit (IMU) that senses the patient's head movement.

In addition, the vestibular rehabilitation training performing device 20 may include a storage unit 230, a communication unit 240, a training progress unit 250, and a score calculation unit 260.

The storage unit 230 may store at least one vestibular rehabilitation training content and at least one computer program configured to perform the vestibular rehabilitation training method.

The vestibular rehabilitation training content may include at least one of vestibular ocular reflex exercise training content, active eye-head exercise training content, target imagination exercise training content, and focusing exercise training content.

Referring back to FIG. 2, in Step S20, the communication unit 240 may receive the training setting information from the vestibular rehabilitation training control device 10. The training progress unit 250 may output the vestibular rehabilitation training content based on the training setting information. The vestibular rehabilitation training content may be displayed on the display unit 200.

While vestibular rehabilitation training content is in progress, the eye tracker 210 may capture the patient's eyeballs to track the patient's gaze, and an IMU sensor 220 may sense the patient's head movements.

An eye tracker 210 may track the patient's gaze using infrared images taken of the eyeball.

The training progress unit 250 may generate position information of a gaze point according to time for the patient's gaze using the patient's gaze data tracked by the eye tracker 210, and may generate head movement information of the patient according to time using the data sensed by the IMU sensor 220. The position information of the gaze point may include a three-dimensional coordinate value where the gaze point is located in a virtual space, and the head movement information may include a direction in which the front of the face faces, a rotation direction of the head, a rotation angle (yaw, pitch, roll), or the like. The training progress unit 250 may proceed with the vestibular rehabilitation training content using the position information of the gaze point and the head movement information.

The technology of generating location information of the gaze point by tracking the patient's gaze using the eye tracker 210 and generating information on the patient's head movement using the IMU sensor 220 is widely known among those skilled in the art, so a detailed description is omitted.

In cases where the eyelids are droopy, such as in the elderly, and the eyeball is partially obscured, the eye tracker may not be able to accurately track the patient's gaze.

As described in Korean Patent No. 10-2748232, the training progress unit 250 may classify the patient's eye state as open, in the process of closing, or fully closed using the artificial neural network model, and may accurately track the patient's gaze even when the patient's eyes are partially covered to generate location information of the gaze point. Through this, the training progress unit 250 may accurately proceed with the vestibular rehabilitation training content regardless of the individual's physical characteristics.

In addition, the training progress unit 250 may temporally synchronize the position information of the gaze point and the patient's head movement information to determine the degree of coordination between the patient's eye and head movements. Depending on the vestibular rehabilitation training content, the criteria for eye and head movements may vary, and the training progress unit 250 may temporally synchronize the position information of the gaze point and the patient's head movement information to determine whether the patient's eye and head movements meet the criteria for each content.

When the training progress unit 250 progresses vestibular rehabilitation training content, the training progress unit may output the method of the corresponding vestibular rehabilitation training to the display unit 200 or output the method through audio (not illustrated) included in the vestibular rehabilitation training performing device 20.

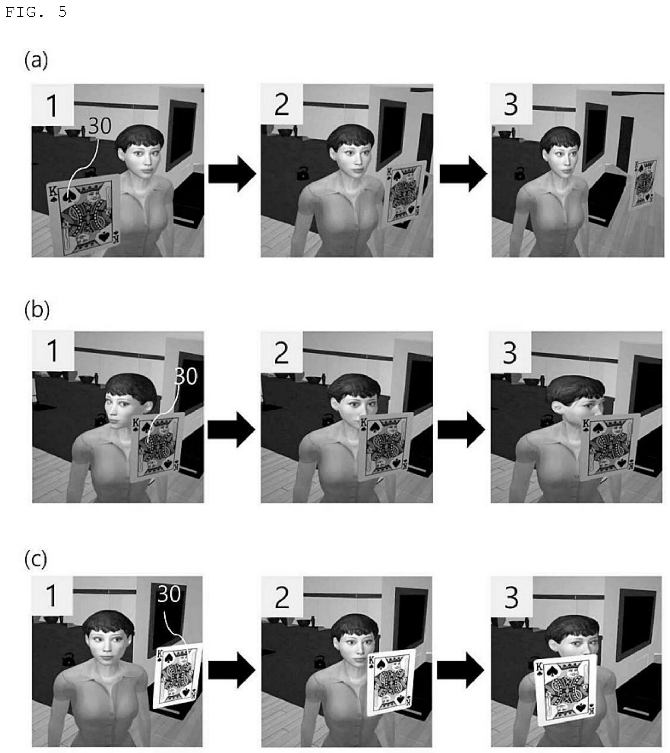

FIGS. 5A to 5C are diagrams illustrating examples of the vestibular ocular reflex exercise training content according to one embodiment of the present disclosure. FIG. 5A is an example of first vestibular ocular reflex exercise training content, FIG. 5B is an example of second vestibular ocular reflex exercise training content, and FIG. 5C is an example of third vestibular ocular reflex exercise training content.

Referring to FIGS. 5A to 5C, in Step S20, the training progress unit 250 may output any one of the first to third vestibular ocular reflex exercise training contents according to the training setting information.

In FIG. 5A, the first vestibular ocular reflex exercise training content may correspond to training content in which a patient moves the patient's eyes toward the virtual target while keeping the patient's head fixed when a virtual target 30 moves up and down and/or left and right at least once.

The training progress unit 250 may output the virtual target 30 having a preset size. The training progress unit 250 may move the virtual target 30 up and down and/or left and right. When the virtual target 30 moves, the training progress unit 250 may determine whether the gaze point of the patient according to the patient's gaze is located in the area occupied by the virtual target 30 in a state where the patient's head is fixed.

The training progress unit 250 may determine that the patient has fixed the head when the change in the rotation angle of the head has a value within a preset range.

When the coordinate values of the gaze point have arbitrary coordinate values within the area occupied by the virtual target 30, the training progress unit 250 may determine that the gaze point is located in the area occupied by the virtual target 30. When the gaze point is located in the area occupied by the virtual target 30, the training progress unit 250 may determine that the patient's gaze is normally directed toward the virtual target 30.

According to one embodiment of the present disclosure, the training progress unit 250 may adjust an error tolerance between the position of the gaze point and the area occupied by the virtual target 30 according to the training difficulty level.

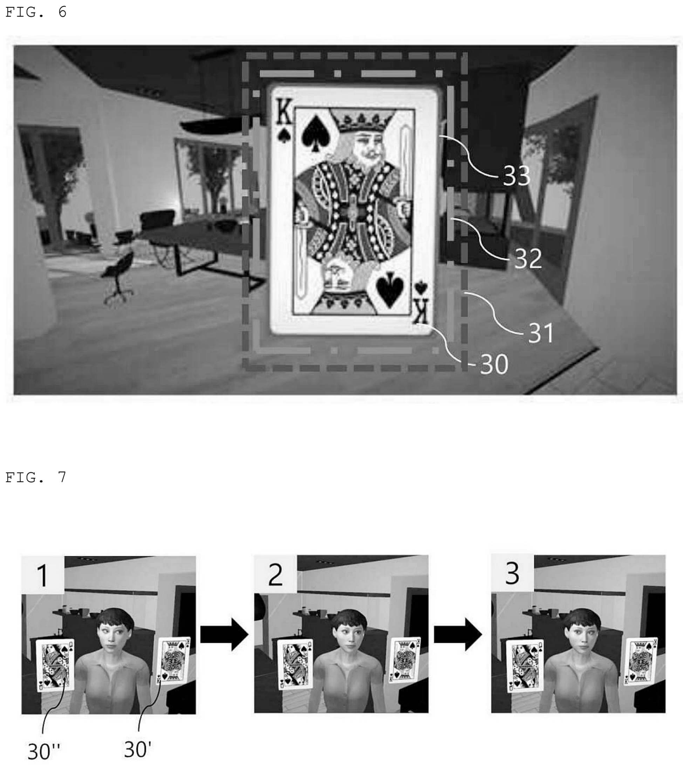

FIG. 6 is a diagram illustrating an example of the error tolerance range according to the training difficulty level.

In the example of FIG. 6, the training progress unit 250 may set the training difficulty level to one of the three levels, from level 1, which has the lowest difficulty level, to level 3, which has the highest difficulty level, according to the training setting information.

When the training difficulty level is the level 1, the training progress unit 250 may determine that the patient's gaze is normally directed toward the virtual target 30 when the gaze point is located within the dotted line (31, red box).

When the training difficulty level is the level 2, the training progress unit 250 may determine that the patient's gaze is normally directed toward the virtual target 30 when the gaze point is located within the dashed line (32, blue box).

When the training difficulty level is the level 3, the training progress unit 250 may determine that the patient's gaze is normally directed toward the virtual target 30 when the gaze point is located within the solid line (33, purple box).

The training progress unit 250 may set a larger error tolerance range between the position of the gaze point and the area occupied by the virtual target 30 as the training difficulty level decreases. The training progress unit 250 may determine that the patient's gaze is directed toward the virtual target 30 when the gaze point is located within the area occupied by the virtual target 30 and the error tolerance range.

Hereinafter, the area occupied by the virtual target 30 and/or the area including the error tolerance range will be referred to as the target fixation area. That is, the target fixation area may mean the area occupied by the virtual target 30 or an area wider than the area occupied by the virtual target 30 by the error tolerance range.

When the gaze point is located in the target fixation area in a state where the patient's head is fixed, this may be considered a clinically normal response. When the gaze point is not located in the target fixation area or the patient moves the head, this may be considered a clinically abnormal response.

In FIG. 5B, the second vestibular ocular reflex exercise training content may correspond to training content in which the patient rotates the patient's head at least once in a state where the patient's gaze is directed toward the virtual target 30 when the virtual target 30 is fixed at an arbitrary position.

In this case, the training progress unit 250 may output sound as audio when the patient turns the patient's head to a preset angle range. When the patient hears the sound, the patient may turn the patient's head in the opposite direction and proceed with the second vestibular ocular reflex exercise training content.

The training progress unit 250 may output the virtual target 30 at an arbitrary location. The training progress unit 250 may determine whether the gaze point is located in the target fixation area when the patient turns the patient's head up and down and/or left and right.

In a case where the gaze point is located in the target fixation area when the patient turns the patient's head through the preset angle range, this may be considered a clinically normal response. When the gaze point is not located in the target fixation area or the patient does not turn the patient's head through the preset angle range, this may be considered a clinically abnormal response.

In FIG. 5C, the third vestibular ocular reflex exercise training content may correspond to training content in which the patient moves the patient's eyes toward the virtual target 30 while turning the patient's head in the opposite direction of the movement of the virtual target 30 when the virtual target 30 moves up and down and/or left and right at least once.

In this case, the training progress unit 250 may output sound as audio when the patient turns the patient's head to a preset angle range. When the patient hears the sound, the patient may turn the patient's head in the opposite direction and proceed with the third vestibular ocular reflex exercise training content.

The training progress unit 250 may determine whether the gaze point is located in the target fixation area when the patient turns the patient's head by a preset angle range in the opposite direction of the movement of the virtual target 30.

In a case where the gaze point is located in the target fixation area when the patient turns the patient's head by the preset angle range, this may be considered a clinically normal response. When the gaze point is not located in the target fixation area or the patient does not turn the patient's head by the preset angle range, this may be considered a clinically abnormal response.

According to one embodiment of the present disclosure, the training progress unit 250 may set the movement speed of the virtual target 30 according to the training difficulty level included in the training setting information. In this case, the training progress unit 250 may set the movement speed of the virtual target 30 to be faster as the training difficulty level increases.

According to one embodiment of the present disclosure, the training progress unit 250 may provide visual feedback when the gaze point is located in the target fixation area. For example, the training progress unit 250 may display a purple border around the virtual target 30 when the gaze point is located in the target fixation area, which is an example and is not limited thereto.

In Step S21, the score calculation unit 260 may calculate a training evaluation score in proportion to the number of times the gaze point is located in the target fixation area.

As described above, the training progress unit 250 may determine whether the patient's eye and head movements meet the criteria for each content by temporally synchronizing the position information of the gaze point and the patient's head movement information. In this case, the training progress unit 250 may determine whether the gaze point is located in the target fixation area according to a preset time interval by using the temporally synchronized position information of the gaze point and the head movement information.

The score calculation unit 260 may count the number of times the gaze point is determined to be located in the target fixation area. The score calculation unit 260 may calculate a higher training evaluation score as the number of times the gaze point is located in the target fixation area increases.

More preferably, the training progress unit 250 may determine whether the reaction is normal or not according to a preset time interval using the position information of the temporally synchronized gaze point and the head movement information. The score calculation unit 260 may calculate a higher training evaluation score as the number of times the reaction is determined to be normal increases.

Additionally, the score calculation unit 260 may calculate a higher training evaluation score as the patient's reaction time is shorter.

In the first vestibular ocular reflex exercise training content, the faster the patient moves the patient's gaze toward the virtual target 30, the shorter the reaction time may be. The faster the patient moves the patient's gaze, the higher the training evaluation score may be calculated.

In the second and third vestibular ocular reflex exercise training content, the faster the patient moves the patient's head in a state where the patient's gaze is directed toward the virtual target 30, the shorter the reaction time may be. The faster the patient moves the patient's head, the higher the training evaluation score may be calculated.

According to another embodiment of the present disclosure, in Step S20, the training progress unit 250 may output the active eye-head movement training content according to the training setting information.

FIG. 7 is a diagram illustrating an example of the active eye-head movement training content.

Referring to FIG. 7, the training progress unit 250 may output two virtual targets 30' and 30" with preset sizes at different locations. The patient may first move the patient's gaze toward either the first virtual target 30' or the second virtual target 30". For convenience, the description will be based on a situation in which the patient first moves the patient's gaze toward the first virtual target 30'.

After the patient moves the patient's gaze toward the first virtual target 30', the patient may turn the patient's head toward the first virtual target 30'. In this case, the training progress unit 250 may determine whether the gaze point is toward the first virtual target 30'. Next, the patient may move the patient's gaze toward the second virtual target 30" and then turn the patient's head toward the second virtual target 30". In this case, the training progress unit 250 may determine whether the gaze point is located in the area occupied by the second virtual target 30".

According to one embodiment of the present disclosure, the training progress unit 250 may set an error tolerance range between the position of the gaze point and the area occupied by each virtual target according to the training difficulty level, and since this has been described above, a repeated description is omitted.

The training progress unit 250 may determine whether the head moves after the gaze point is located within a first target fixation area according to the first virtual target 30' or within a second target fixation area according to the second virtual target 30". More specifically, the training progress unit 250 may calculate a time difference between the time when the gaze point is located within the first target fixation area or the second target fixation area and the time when the head moves. The training progress unit 250 may determine whether the head moves after the patient's gaze moves by using the time difference.

When the patient turns the patient's head toward the first virtual target 30" or the second virtual target 30" in a state where the patient moves the patient's gaze toward the first virtual target 30' or the second virtual target 30", a situation in which the gaze point is located in the target fixation area according to the virtual target may be considered a clinically normal response.

In addition, when the patient turns the patient's head toward the first virtual target 30" or the second virtual target 30" in a state where the patient moves the patient's gaze toward the first virtual target 30' or the second virtual target 30", the gaze point may deviate the target fixation area. In this case, the patient may move the patient's gaze so that the gaze point is located in the target fixation area. A situation in which the gaze point returns to the target fixation area within a preset time may also correspond to a clinically normal response.

When the patient turns the patient's head toward the first virtual target 30' or the second virtual target 30" in a state where the patient moves the patient's gaze toward the first virtual target 30' or the second virtual target 30", a situation in which the gaze point is not located in the target fixation area according to the virtual target or the patient's head turns before the patient's gaze moves toward the virtual target may be a clinically abnormal response.

According to one embodiment of the present disclosure, the training progress unit 250 may provide the visual feedback when the gaze point is located in the target fixation area according to the first virtual target 30' or the second virtual target 30". For example, when the gaze point exists in the first target fixation area, the training progress unit 250 may display the border of the first virtual target 30' in orange.

In addition, the training progress unit 250 may visually display the virtual target differently when the patient's gaze and the frontal direction of the patient's face are directed toward the same virtual target. For example, when the gaze point is located in the first target fixation area, the training progress unit 250 may display the border of the first virtual target 30' in orange. Thereafter, when the frontal direction of the patient's face is directed toward the first virtual target 30', the training progress unit 250 may display the border of the first virtual target 30' in purple.

In Step S21, the score calculation unit 260 may calculate a return time, which is the time taken for the gaze point to move back to the target fixation area after deviating the target fixation area according to the virtual target when the patient turns the patient's head toward the first virtual target 30' or the second virtual target.

More specifically, the patient may turn the front of the face toward the first virtual target 30' in a state where the patient's gaze is directed toward the first virtual target 30'. In this case, the patient's gaze may deviate from the first target fixation area due to inertia according to the head rotation, and the patient may move the gaze toward the first target fixation area again. In this process, the gaze point may return to the first target fixation area again after the gaze point deviates from the first target fixation area.

The score calculation unit 260 calculates the return time, and the shorter the return time, the higher the training evaluation score may be calculated.

More preferably, the score calculation unit 260 may calculate the return time in the case of the normal reaction, and calculate a higher training evaluation score as the return time is shorter.

In addition, the score calculation unit 260 may calculate a higher training evaluation score as the patient's reaction time is shorter. In the active eye-head movement training content, the faster the patient moves the patient's gaze and face front toward the first virtual target 30' or the second virtual target 30", the shorter the reaction time may be. The shorter the patient's reaction time, the higher the training evaluation score may be calculated.

According to still another embodiment of the present disclosure, in Step S20, the training progress unit 250 may output the target imaginary exercise training content according to the training setting information.

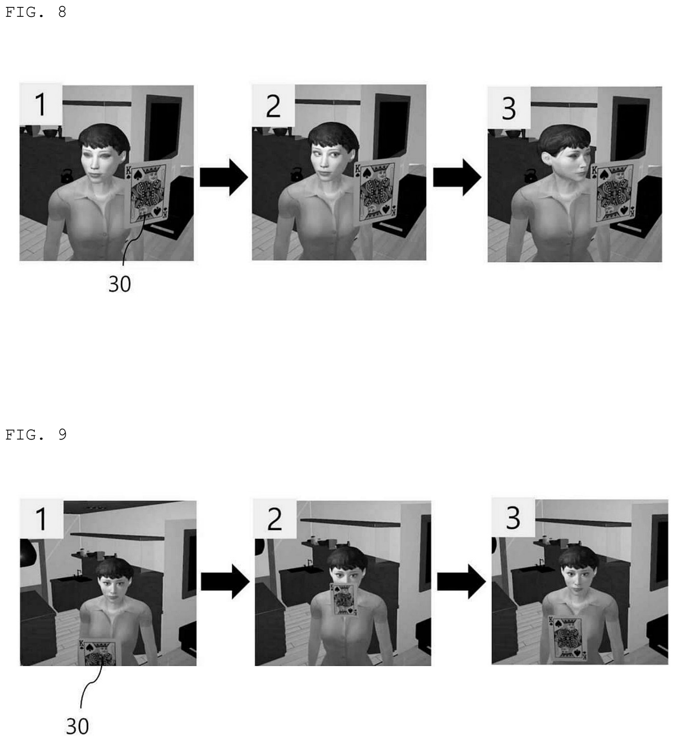

FIG. 8 is a diagram illustrating an example of the target imagination exercise training content.

Referring to FIG. 8, the training progress unit 250 may output the virtual target 30 fixed at an arbitrary location. The patient may close the patient's eyes and turn the patient's head in a state where the patient's gaze is directed toward the virtual target 30. The training progress unit 250 may output a sound through audio, and the patient may open the patient's eyes again when the patient hears the sound. The training progress unit 250 may determine whether the patient's eyes are closed or open according to the method described in Korean Patent No. 10-2748232. The training progress unit 250 may determine whether the gaze point is located at the virtual target 30 when the patient opens the patient's eyes again.

According to one embodiment of the present disclosure, the training progress unit 250 may set the error tolerance range between the position of the gaze point and the area occupied by the virtual target 30 according to the training difficulty level, and since this has been described above, a repeated description thereof is omitted.

In a case where the patient rotates the patient's head by a preset angle range in a state of closing the patient's eyes and the gaze point is located in the target fixation area when the patient opens the patient's eyes again, this may be considered a clinically normal response. When the gaze point is not located in the target fixation area, or the patient does not turn the patient's head by the preset angle range, this may be considered a clinically abnormal response.

The training progress unit 250 may provide the visual feedback in a case where the gaze point is located within the target fixation area when the patient opens the patient's eyes again. For example, the training progress unit 250 may display the border of the virtual target 30 in purple in a case where the gaze point is located within the target fixation area when the patient opens the patient's eyes again.

In Step S21, the score calculation unit 260 may calculate the training evaluation score inversely proportional to the degree to which the gaze point deviates from the target fixation area according to the virtual target 30. The score calculation unit 260 may calculate a higher training evaluation score the closer the gaze point is to the target fixation area when the patient opens the patient's eyes again.

More preferably, the score calculation unit 260 may calculate a higher training evaluation score the closer the gaze point is to the target fixation area in the case of the normal reaction.

In addition, the score calculation unit 260 may calculate a higher training evaluation score as the patient's reaction time is shorter. In the target imaginary exercise training content, the faster the patient turns the patient's head with the patient's eyes closed, or the faster the patient opens the patient's eyes upon hearing a sound, the shorter the reaction time may be. The shorter the patient's reaction time, the higher the training evaluation score may be calculated.

According to still another embodiment of the present disclosure, in Step S20, the training progress unit 250 may progress the focusing exercise training content according to the training setting information.

FIG. 9 is a diagram illustrating an example of the focusing exercise training content.

Referring to FIG. 9, the training progress unit 250 may output the virtual target 30 and move the virtual target 30 away from or closer to the patient's field of vision. When the virtual target 30 moves away from or closer to the patient's field of vision, the patient may maintain the patient's gaze toward the virtual target 30.

According to one embodiment of the present disclosure, the training progress unit 250 may set an error tolerance range between the position of the gaze point and the area occupied by the virtual target 30 according to the training difficulty level, and since this has been described above, the repeated description thereof is omitted.

Additionally, the training progress unit 250 may adjust the movement speed of the virtual target 30 according to the training difficulty level.

In Step S21, the score calculation unit 260 may calculate a higher training evaluation score as the number of data points in which the gaze point is located within the target fixation area increases.

Thereafter, the communication unit 240 may transmit the calculated training evaluation score to the vestibular rehabilitation training control device 10. The vestibular rehabilitation training control device 10 may store and manage the patient's training evaluation score.

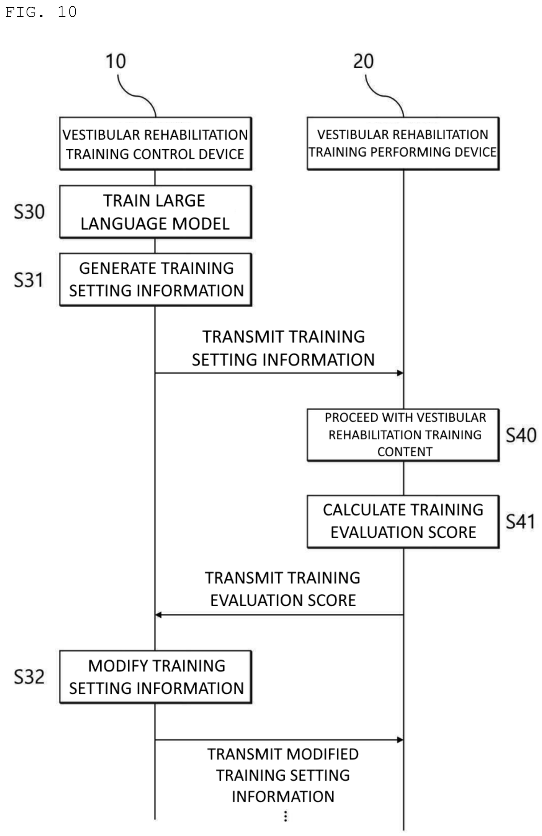

FIG. 10 is a flowchart of the vestibular rehabilitation training control method and the vestibular rehabilitation training performing method according to another embodiment of the present disclosure.

Referring to FIG. 10, Steps S30 and S31 are identical to Steps S10 and S11, and Steps S40 and S41 are identical to Steps S20 and S21, so the repeated description thereof is omitted.

In Step S32, the processor may receive the training evaluation score from the vestibular rehabilitation training performing device 20. The processor may modify the training setting information based on the training evaluation score.

According to one embodiment of the present disclosure, when the training evaluation score is equal to or less than a preset first reference value, the processor may set the training difficulty level included in the training setting information generated in Step S30 to be relatively low. The first reference value may correspond to a criterion for determining whether the patient is performing vestibular rehabilitation training normally. A low training evaluation score may mean that the patient may not accurately gaze at the virtual target or have a relatively long reaction time.

In addition, the processor may set the training difficulty level included in the training setting information generated in Step S30 relatively high when the training evaluation score is equal to or more than a preset second reference value. The second reference value may correspond to a criterion for determining whether the set training difficulty level is an easy difficulty for the patient. A high training evaluation score may mean that the patient accurately gazes at the virtual target or has a relatively short reaction time.

The above processor may transmit the modified training setting information to the vestibular rehabilitation training performing device 20. The vestibular rehabilitation training performing device 20 may proceed with the vestibular rehabilitation training content according to the modified training setting information.

By adjusting the difficulty level of vestibular rehabilitation training according to the training evaluation score, a vestibular rehabilitation training system 1 may provide personalized vestibular rehabilitation training content to the patient.

In the above, it has been described that after the vestibular rehabilitation training content is finished, the vestibular rehabilitation training performing device 20 transmits the calculated training evaluation score to the vestibular rehabilitation training control device 10 to adjust the training difficulty level. However, this is only an example, and the vestibular rehabilitation training performing device 20 may transmit rehabilitation training data to the vestibular rehabilitation training control device 10 in real time. The vestibular rehabilitation training control device 10 may adjust the training difficulty level in real time based on the rehabilitation training data acquired in real time and transmit the training data to the vestibular rehabilitation training performing device 20. Through this, the vestibular rehabilitation training performing device 20 may adjust the training difficulty level in real time to provide the patient with vestibular rehabilitation training content customized for the patient.

The vestibular rehabilitation training control device 10 may further include an artificial neural network model that calculates training difficulty level by using the patient's rehabilitation training data as input values. The processor may input the rehabilitation training data received in real time into the artificial neural network model to obtain the training difficulty level information and transmit the training difficulty level information to the vestibular rehabilitation training performing device 20.

The storage unit 230, communication unit 240, training progress unit 250, and score calculation unit 260 may include a processor, an application-specific integrated circuit (ASIC), another chipset, a logic circuit, a register, a communication modem, a data processing device, or the like known in the technical field to which the present disclosure belongs in order to execute calculation and various control logics. In addition, when the above-described control logic is implemented as software, the storage unit 230, communication unit 240, training progress unit 250, and score calculation unit 260 may be implemented as a set of program modules. In this case, the program modules may be stored in the memory device and executed by the processor.

The vestibular rehabilitation training control method and the vestibular rehabilitation training performing method may be implemented in the form of a computer program prepared to perform each step on a computer and recorded on a computer-readable recording medium. The above-mentioned computer program may include codes coded in a computer language, such as C/C++, C#, JAVA, Python, or machine language, that may be read by the processor (CPU) of the computer through the device interface of the computer, so that the computer reads the program and executes the methods implemented as the program. Such codes may include functional codes related to functions that define functions necessary for executing the methods, and may include control codes related to execution procedures necessary for the processor of the computer to execute the functions according to a predetermined procedure by means of processor of the computer. In addition, such codes may further include memory reference-related codes regarding which location (address number) of the internal or external memory of the computer should be referenced for additional information or media necessary for the processor of the computer to execute the functions. In addition, when the processor of the computer needs to communicate with another computer or server located remotely in order to execute the functions, the code may further include communication-related code regarding how to communicate with another computer or server located remotely using the communication module of the computer, what information or media to send and receive during communication, or the like.

The above storage medium means a medium that stores data semi-permanently and may be read by a device, rather than a medium that stores data for a short period of time, such as a register, cache, or memory. Specifically, examples of the storage medium include, but are not limited to, ROM, RAM, CD-ROM, magnetic tape, floppy disk, or optical data storage device. That is, the program may be stored in various storage media on various servers that the computer may access or in various storage media on the user's computer. In addition, the medium may be distributed to computer systems connected to a network, so that a computer-readable code may be stored in a distributed manner.

Although the embodiments of the present disclosure have been described above with reference to the attached drawings, it will be understood by those skilled in the art to which the present disclosure pertains that the present disclosure may be implemented in other specific forms without changing the technical idea or essential characteristics thereof. Therefore, it should be understood that the embodiments described above are illustrative in all respects and not restrictive.

## Claims

1. A vestibular rehabilitation training control device, comprising:
a hardware processor; and
a storage unit connected to the processor and storing a database storing questionnaire information, clinical information, and rehabilitation training information of a plurality of patients as a data set, and storing at least one computer program configured to perform a vestibular rehabilitation training control method,
wherein the vestibular rehabilitation training control method includes
(a) training a large language model to generate training setting information by receiving the questionnaire information using the questionnaire information, clinical information, and rehabilitation training information of the plurality of patients as learning data,
(b) comparing the questionnaire information input from the patient with a plurality of data sets stored in the database to generate the training setting information or inputting the questionnaire information to the large language model to obtain the training setting information, and
(c) transmitting the training setting information to a vestibular rehabilitation training performing device.

2. The vestibular rehabilitation training control device of claim 1, wherein the questionnaire information includes Dizziness Handicap Inventory (DHI) and Vestibular Disorders Activities of Daily Living Scale (VADL) questionnaire information.

3. The vestibular rehabilitation training control device of claim 1, wherein the (b) includes
generating, when a data set having the same questionnaire information as the questionnaire information input from the patient exists in the database, the training setting information based on rehabilitation training information of the data set, and
inputting, when the data set having the same questionnaire information as the questionnaire information input from the patient does not exist in the database, the questionnaire information into the large language model to obtain the training setting information.

4. The vestibular rehabilitation training control device of claim 1, wherein the training setting information includes at least one of a vestibular rehabilitation training content type and a training difficulty level.

5. The vestibular rehabilitation training control device of claim 1, wherein the vestibular rehabilitation training control method further includes (d) modifying the training setting information based on a training evaluation score received from the vestibular rehabilitation training performing device.

6. The vestibular rehabilitation training control device of claim 5, wherein the (d) includes setting the training difficulty level included in the training setting information to be relatively low when the training evaluation score is equal to or less than a preset reference value.

7. A vestibular rehabilitation training performing device comprising:
a housing having a preset shape, and including a display unit for displaying vestibular rehabilitation training content, an eye tracker for tracking the gaze of a patient, and an Inertial Measurement Unit (IMU) sensor for sensing a head movement of the patient;
a storage unit for storing at least one vestibular rehabilitation training content and at least one computer program configured to perform a vestibular rehabilitation training performing method;
a communication unit for receiving training setting information from a vestibular rehabilitation training control device and transmitting a training evaluation score to the vestibular rehabilitation training control device;
a training progress unit for outputting the vestibular rehabilitation training content based on the training setting information received from the vestibular rehabilitation training control device, generating position information of a gaze point according to the gaze of the patient using data tracked by the eye tracker, and generating head movement information of the patient using data sensed by the IMU sensor to perform the vestibular rehabilitation training content; and
a score calculation unit for calculating the training evaluation score for the vestibular rehabilitation training content using the position information of the gaze point and the head movement information.

8. The vestibular rehabilitation training performing device of claim 7, wherein the at least one vestibular rehabilitation training content includes at least one of vestibular ocular reflex exercise training content, active eye-head exercise training content, and target imagination exercise training content.

9. The vestibular rehabilitation training performing device of claim 8, wherein when the vestibular rehabilitation training content is vestibular ocular reflex exercise training content,
the training progress unit outputs a virtual target having a preset size and fixed or moving at an arbitrary location, and determines whether the gaze point is located in an area occupied by the virtual target when the patient fixes or moves the head.

10. The vestibular rehabilitation training performing device of claim 9, wherein the score calculation unit calculates the training evaluation score in proportion to the number of times the gaze point is located in the area occupied by the virtual target.

11. The vestibular rehabilitation training performing device of claim 8, wherein when the vestibular rehabilitation training content is the active eye-head movement training content,
the training progress unit outputs two virtual targets having preset sizes at different locations, and when the patient moves the patient's gaze toward a first virtual target and then turns the patient's head toward the first virtual target, the training progress unit determines whether the gaze point is located in an area occupied by the first virtual target, and when the patient moves the patient's gaze toward a second virtual target and then turns the patient's head toward the second virtual target, the training progress unit determines whether the gaze point is located in an area occupied by the second virtual target.

12. The vestibular rehabilitation training performing device of claim 11, wherein when the patient turns the patient's head toward the first virtual target or the second virtual target, the score calculation unit calculates the training evaluation score in inverse proportion to a time taken for the gaze point to move back to the area occupied by the virtual target after deviating the area occupied by the virtual target.

13. The vestibular rehabilitation training performing device of claim 8, wherein when the vestibular rehabilitation training content is the target imagination exercise training content,
the training progress unit outputs a virtual target having a preset size and fixed at an arbitrary location, and when the patient closes the patient's eyes in a state where the patient's gaze is directed toward the virtual target and the patient opens the patient's eyes after turning the patient's head, the training progress unit determines whether the gaze point is located in the area occupied by the virtual target.

14. The vestibular rehabilitation training performing device of claim 13, wherein the score calculation unit calculates the training evaluation score in inverse proportion to an extent to which the gaze point deviates from the area occupied by the virtual target.

15. The vestibular rehabilitation training performing device of claim 7, wherein the training progress unit provides visual feedback when the gaze point is located in an area where at least one virtual target output from the vestibular rehabilitation training content is located.

16. A vestibular rehabilitation training system comprising:
the vestibular rehabilitation training control device according to any one of claims 1; and
the vestibular rehabilitation training performing device according to any one of claims 7.

17. A computer program prepared to perform each step of the vestibular rehabilitation training control method according to any one of claims 1 on a computer and recorded on a computer-readable recording medium.
